# EUROPEAN PATENT APPLICATION

(11) **EP 1 721 524 A1**
(43) Date of publication of application: **15.11.2006**
(21) Application number: 05719524.0
(22) Date of filing: 25.02.2005
(51) Int. Cl.: A01N 65/00, A01N 43/16, A23L 3/3544, A61K 8/18, A61K 36/18, A61P 31/00, C07D 311/58, C07D 311/64

(54) **ANTIBACTERIAL AGENT AND ANTIBACTERIAL COMPOSITION**

(30) Priority: 27.02.2004 JP 2004054936
(71) Applicant: Sakamoto Bio Co. Ltd., Akita 0101233 (JP)
(72) Inventor: SAKAMOTO, Kenji Sakamoto Bio Co., Ltd., Akita-shi Akita 0101233 (JP); MUKAIYAMA, Toshiyuki Sakamoto Bio Co., Ltd., Akita-shi Akita 0101233 (JP); HORI, Kazuyuki Akita Research Inst. Food & Brewing, Akita-shi Akita 0101623 (JP); TAKAHASHI, S. Akita Research Inst. Food & Brewing, Akita-shi Akita 0101623 (JP)
(74) Representative: Fisher, Adrian John
(86) International application number: PCT/JP2005/003123
(87) International publication number: WO 2005/082151

(57) **Abstract**

[Problem] The problems to be solved by the invention is to provide antibacterial agents originated from natural products that are safe for regular use with potent antibacterial activity, and antibacterial compositions containing said antibacterial agents.

[Solution] Antibacterial agents containing Eysenhardtia adenostylis extract or isoflavan compounds. Also, antibacterial agents containing Eysenhardtia adenostylis extract or isoflavan compounds as active ingredients. Said antibacterial agents can be applied to products such as cosmetics, including quasi-drugs, pharmaceuticals and foods. Further, antibacterial compositions containing said antibacterial agents, Eysenhardtia adenostylis extract or isoflavan compounds, and said compositions include cosmetics, including quasi-drugs, pharmaceuticals and foods.

## Description

### Field of the Invention

The present invention relates to the identification of antibacterial agents with significant antibacterial properties that can function to prevent contamination by microorganisms such as bacteria and fungi, but it also relates to the use of these antibacterial agents in commercial products such as cosmetics, pharmaceuticals and foods.

### Background Technology

Various antibacterial and disinfecting agents are conventionally used in the food and cosmetic industries to prevent product contamination by microorganisms and to enhance the shelf-life and safety of products. Also, a variety of disinfectants and antiseptics are used in the cosmetic and pharmaceutical industries to prevent skin diseases caused by microorganisms, and to improve complexion. For example, antibacterial agents such as paraben and triclosan are widely used in the cosmetic and food sectors.

Antibacterial and disinfecting agents often cannot be admixed in amounts sufficiently large so as to exhibit actual antibacterial effects, because the amounts to be admixed are limited from the safety point of view by the Japanese Pharmacopoeia, the Japanese Standards of Cosmetic Ingredients and the Japanese Standards of Food Additives. Therefore, antibacterial agents from natural sources with high antibacterial activity have been developed, for example, tansy extract and green tea extract (see, for example, Patent literature 1). Moreover many essential oils (see, for example, Patent literature 2), sorbic acid ester (see, for example, Patent literature 3), saponin (see, for example, Patent literature 4, 5) are widely used. But the antibacterial capacity of all of these, however, is insufficient. Thus the development of safe and effective antibacterial agents is still desired.

The plant relating to the present invention, whose academic name is Eysenhardtia adenostylis, is known for its use to cure renal diseases through use of an extract, the green liquid obtained by immersing its stem or bark in water. However the antibacterial effect of this extract until now has been unknown. Also, amorphaquinone, mucronulatol, lonchocarpin and pendulone are known substances [amorphaquinone (see Non-patent literature 1), mucronulatol (see Non-patent literature 2, 3), lonchocarpin (see Non-patent literature 4), pendulone (see Non-patent literature 5)], whose antibacterial effect, however, is unknown.

Patent literature 1: Japanese Published Unexamined Application Hei No. 11-228325
Patent literature 2: Japanese Published Unexamined Application No. 2001-64163
Patent literature 3: Japanese Published Unexamined Application No. 2000-256107
Patent literature 4: Japanese Published Unexamined Application Hei No. 7-149608
Patent literature 5: Japanese Published Unexamined Application No. 2001-39812
Non-patent literature 1: Shibata, H. and Shimizu, S. 1978. Heterocycles 10: 85-86.
Non-patent literature 2: Kurosawa, K. 1978. Phytochemistry 17: 1385, 1405.
Non-patent literature 3: Humburger, M.O. 1987. Journal of Natural Products 50: 696.
Non-patent literature 4: Pelter, A. 1969. Journal of Chemical Society: 887.
Non-patent literature 5: Hayashi, Y. 1978. Journal of Japan Wood Research Society 24: 8, 98.

### Disclosure of Invention

### Problems to be Solved by the Invention

The present invention is accomplished in the light of the above-mentioned facts. The object of the invention is to provide effective antibacterial agents that originate from natural products for commercial applications. The aim is to identify an agent which is safe for regular use and which has high antibacterial activity, and the further aim is the creation of safe antibacterial products containing said agent.

### Means for Solving the Problem

As a result of careful research to solve the above-mentioned problems, the inventors found that Eysenhardtia adenostylis extract had a high antibacterial activity, and discovered that the active ingredients contained in it were specifically structured isoflavan compounds, and as a result they made the present invention.

Thus, the present invention provides antibacterial agents comprising Eysenhardtia adenostylis extract.

The invention also provides antibacterial agents comprising Eysenhardtia adenostylis extract specifically for use as active ingredients.

The invention also provides antibacterial agents comprising

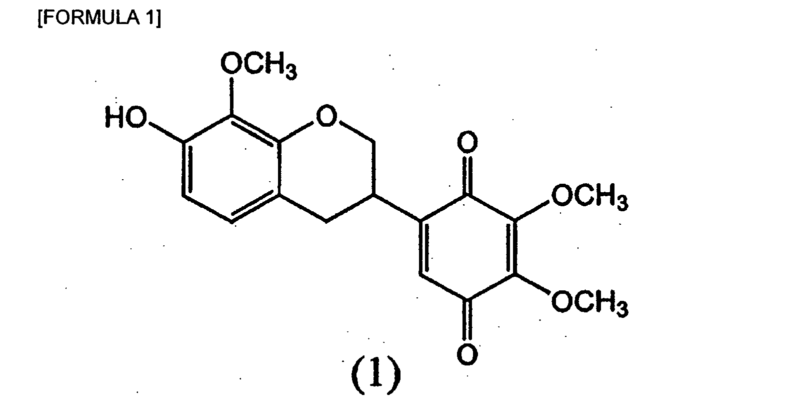

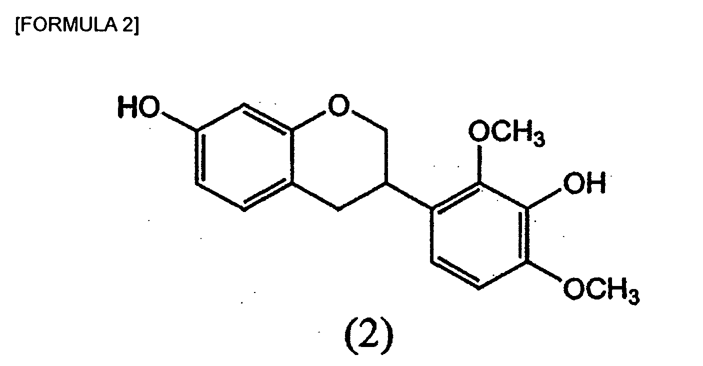

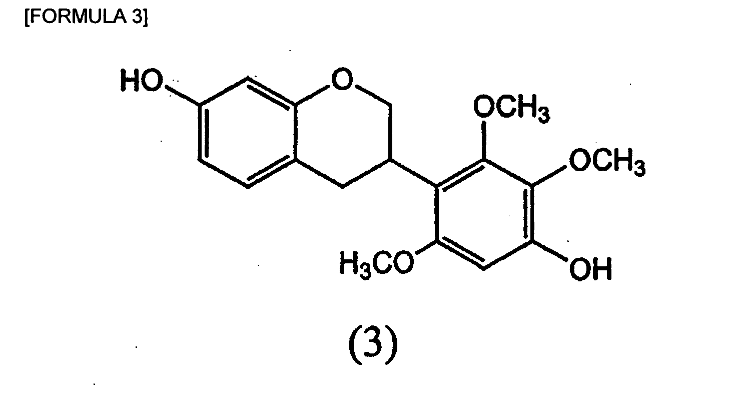

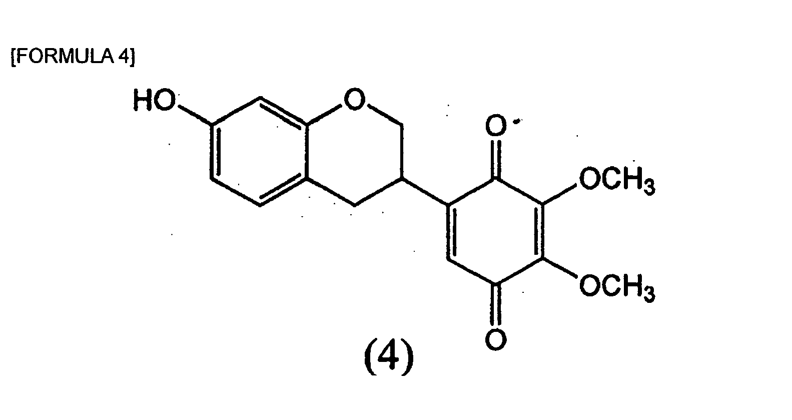

that is, one or more compounds selected from the isoflavan compounds shown in the above formulae (1), (2), (3) and (4).

The invention also provides antibacterial agents comprising one or more compounds selected from the isoflavan compounds shown in the above formulae (1), (2), (3) and (4) as active ingredients.

Additionally, the invention provides antibacterial compositions containing the above antibacterial agents.

The invention also provides design of antibacteiral compositions containing said Eysenhardtia adenostylis extract.

The invention also provides antibacterial compositions containing one or more compounds selected from the isoflavan compounds shown in the above formulae (1), (2), (3) and (4).

These antibacterial compositions can be cosmetics, pharmaceuticals or foods.

Further, the invention provides compositions containing the above antibacterial agents.
Said compositions can be cosmetics, pharmaceuticals or foods.

The invention also provides compositions containing the Eysenhardtia adenostylis extract. Said compositions can be cosmetics, pharmaceuticals or foods.

The invention also provides compositions containing one or more compounds selected from the isoflavan compounds shown in the above formulae (1), (2), (3) and (4). Said compositions can be cosmetics, pharmaceuticals or foods.

The term "antibacterial agents" as used in the present invention, includes germicides, antiseptics, preservatives, antibacterials and the like.

### Effect of the Invention

According to the present invention, antibacterial agents which are safe for regular use were obtained by finding that Eysenhardtia adenostylis extract and isoflavan compounds of a specific structure existing in Eysenhardtia adenostylis have antibacterial activity. The antibacterial agents of the invention have potent antibacterial activity and a wide range of applications. The antibacterial agents of the invention are mixed into compositions as antibacterial components, and form new and effective compositions with antibacterial activity. Thus there are antibacterial compositions, which can be used to prevent diseases caused by microorganisms, and to treat said diseases. The antibacterial agents of the invention, added to products that require antibacterial and antiseptic properties, such as cosmetics, pharmaceuticals and foods, exhibit antibacterial activity, which can effectively suppress proliferation of bacteria and fungi, and prevent microorganism contamination by achieving their antibacterial and antifungal activity.

### Best Mode for Carrying out the Invention

The mode for carrying out the present invention is hereinafter explained in detail.

The Eysenhardtia adenostylis extract relating to the present invention can be obtained by a common extraction method using Eysenhardtia adenostylis, which is a method for extracting Eysenhardtia adenostylis into solvents.

The plant relating to the invention, whose scientific name is Eysenhardtia adenostylis, is a papilionaceous shrubby plant, which is called Taray in some parts of Central America.

As for parts of Eysenhardtia adenostylis which can be subjected to extraction, the entire plant, xylem, barks, heartwood, branches, leaves, stems, pods, roots, flowers, seeds and the like are usable. Particularly, it is preferable to use xylem and roots in consideration of effectiveness and efficiency of extraction. As for forms of Eysenhardtia adenostylis used for extraction, various forms, for example, fresh, fresh and cut into pieces, dried, dried and cut into pieces or powdered, and the like are used, however the forms of Eysenhardtia adenostylis used for extraction are not limited to these; other forms can be used within the range where the effects of the present invention are not blocked. In this invention, it is desirable to carry out solvent extraction after transforming Eysenhardtia adenostylis into a dried, powder or other form, which are the most efficient forms for extraction.

Though there is no particular limitation, solvents for extraction include, for example, alcohols such as ethanol, methanol, isopropanol and 1,3-butylene glycol; hydrocarbons such as hexane, heptane and cyclohexane; esters such as ethyl acetate; ketones such as acetone; and water, solo or any two or more of which can be used in combination. Of these solvents, ethanol, methanol or a mixed solvent of these are preferable for producing the effects of the present invention.

Methods such as solvent soak and solvent reflux are used for extraction. As for extraction temperature, there is no particular limitation thereto; extraction is performed at room temperature and the boiling point of a solvent under normal atmospheric pressure. There is no particular limitation to extraction time, wherein extraction is performed for any length of time. After solvent extraction, Eysenhardtia adenostylis extract is obtained by removing extraction remainder such as solids with methods such as filtration and centrifugation, and then by removing the extraction solvent from the extract.

The compound shown in the following Formula (1),

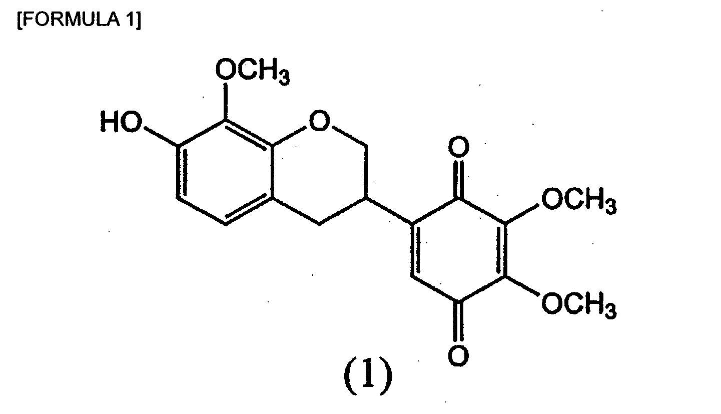

which relates to the present invention, is amorphaquinone, the compound shown in the following Formula (2)

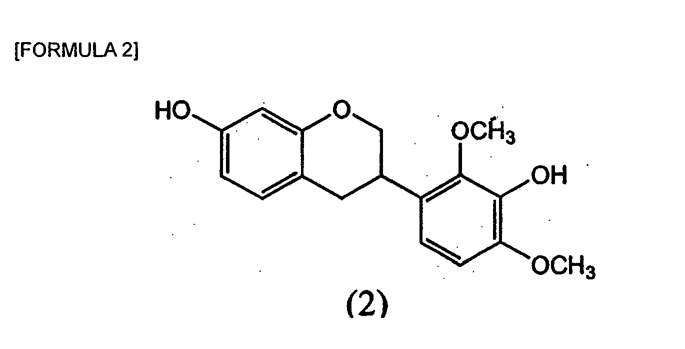

is mucronulatol, the compound shown in the following Formula (3)

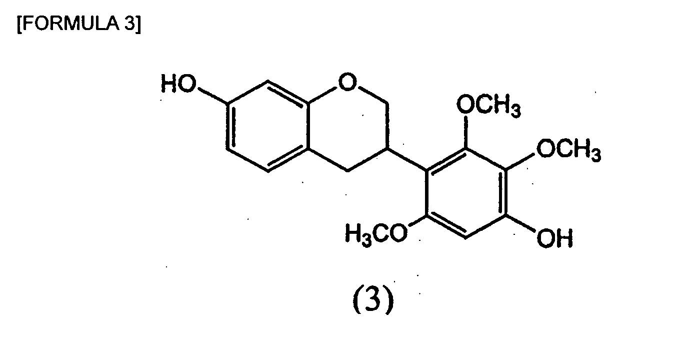

is lonchocarpin, and the compound shown in the following Formula (4)

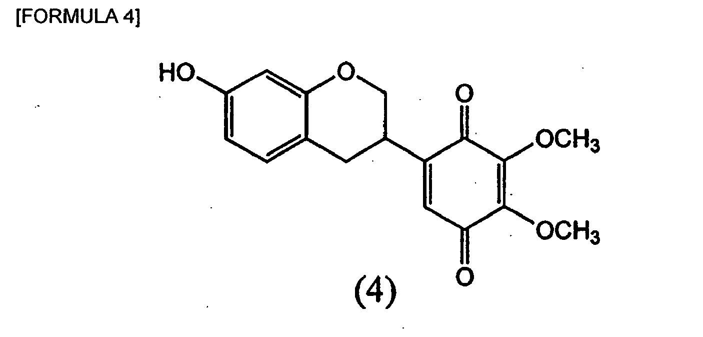

is pendulone, all of which are isoflavan compounds. Any of the isoflavan compounds shown in the above formulae (1), (2), (3) and (4) are components existing in Eysenhardtia adenostylis and can be extracted from said Eysenhardtia adenostylis. It was found for the first time by the present inventors that the isoflavan compounds shown in the above formulae (1), (2), (3) and (4) exist in Eysenhardtia adenostylis. A heretofore known isolation method can be used to isolate isoflavan compounds shown in the above formulae (1), (2), (3) and (4) from Eysenhardtia adenostylis extract.

For example, from Eysenhardtia adenostylis extract, the isoflavan compounds shown in the above formulae can be isolated and obtained through repeating fractionation such as column chromatographies, but not limited to these.

Eysenhardtia adenostylis extract used for isolating the isoflavan compounds shown in the above formulae (1), (2), (3) and (4) can be used not only as dry extract, which is obtained by removing solvents from solvent extract of Eysenhardtia adenostylis, thus in the form of extract, but also in the form of liquefied extract obtained by extraction using a solvent; further, Eysenhardtia adenostylis extract can be used as extract dilution, which is obtained by diluting the extract with the solvent, and as extract concentrate, which is obtained by removing any amount of the solvent and concentrating the extract. Moreover, the extract can be used after additional processes such as deodorization and decolorization within the range where the effects of the present invention are not spoiled.

The isoflavan compounds shown in formulae (1), (2), (3) and (4) used in the present invention can be obtained by, in addition to said extraction from Eysenhardtia adenostylis, for example, isolation from plants other than Eysenhardtia adenostylis which comprise any of the isoflavan compounds shown in the formulae (1), (2), (3) and (4). Also the compounds can be obtained by synthesis.

The Eysenhardtia adenostylis extract relating to the present invention and the isoflavan compounds shown in formulae (1), (2), (3) and (4) both have potent antibacterial activity as described hereafter. Thus, Eysenhardtia adenostylis extract or one or more types of compounds (Hereinafter, one or more types of compounds selected from the isoflavan compounds shown in formulae (1), (2), (3) and (4) will be simply called the "isoflavan compounds.") selected from the isoflavan compounds shown in the above formulae (1), (2), (3) and (4) are useful as antibacterial agents.

Moreover, they are useful as antibacterial agents containing Eysenhardtia adenostylis extract or the isoflavan compounds as active ingredients. These antibacterial agents provide a new and novel use based on the finding of said new function of Eysenhardtia adenostylis extract and the isoflavan compounds.

The antibacterial agents of the invention have an extremely broad range of applications, which can be used in different industry sectors. Said sectors include, for example, cosmetics including quasi-drugs, pharmaceuticals and foods, which are most suitable. As for application of the antibacterial agents of the present invention, any forms such as powders, granules and emulsions can be used according to the use. Also, the agents can be used together with other antibacterial agents, preservatives and the like, in which case they have a combined effect with the antibacterial agents of the invention. There is no particular limitation to the materials to be used together, which include, for example, botanical extract which has antibacterial activity, benzoate or salt thereof, orthophenylphenol or salt thereof, diphenyl, sorbic acid or salt thereof, dehydroacetic acid or salt thereof, p-hydroxybenzoate, p-hydroxybenzoic alkyl esters (methyl parahydroxybenzoate, ethyl parahydroxybenzoate, propyl parahydroxybenzoate and the like), propionic acid or salt thereof, chlorhexidine gluconate and the like.

As for the forms of Eysenhardtia adenostylis extract and isoflavan compounds used for the application of the antibacterial agents of the present invention, Eysenhardtia adenostylis extract can be used not only in the form of dry extract which is obtained by removing a solvent from a solvent extract of Eysenhardtia adenostylis, thus extract, but also in various forms; for example, in the form of liquefied extract obtained by extraction using a solvent, and also in the form of extract dilution, which is obtained by diluting the extract with the solvent, and in the form of extract concentrate, which is obtained by removing any amount of the solvent and concentrating the extract. Also, the extract can be used after additional processes such as deodorization (deodorized form) and refinement (refined form) if they do not impair the effects of the present invention. It can be also used as dry extract solution obtained by dissolving dry extract in a solvent at any concentration. Moreover, it can be used as fraction or even active ingredients by refining it using column chromatography and the like.

Also, isoflavan compounds can be used not only as said compounds singly, but also as plants or in the form of extract thereof which comprise isoflavan compounds as active ingredients and comprise isoflavan conpounds at a concentration where antibacterial activity (function) is effectively performed. The forms of said extract can be solvent extract, concentration solution thereof, and even any fractions containing isoflavan compounds obtained by refining the extract if the condition that said antibacterial activity is effectively performed is satisfied.

The antibacterial agents relating to the present invention, or Eysenhardtia adenostyiis extract or isoflavan compounds as antibacterial agents are mixed into compositions as antibacterial ingredients, and prepared as new and effective compositions with antibacterial activity, thus antibacterial compositions. Therefore, compositions, which contain the antibacterial agents relating to the present invention, Eysenhardtia adenostylis extract or isoflavan compounds, are useful as antibacterial compositions which has antibacterial effect. Said antibacterial compositions of the present invention, in the compositions, can exhibit various antibacterial activities, for example, effects that prevent growth of microorganisms such as bacteria and fungi and preserve compositions from microorganism contamination, and medicinal benefits that improve and prevent diseases caused by microorganisms. Though there is no particular limitation, said compositions include, for example, cosmetics including quasi-drugs, pharmaceuticals and foods, which are most suitable. Particularly, they are suitably used as cosmetics.

When the antibacterial agents relating to the present invention, Eysenhardtia adenostylis extract or isoflavan compounds are mixed with said antibacterial compositions, the content of the antibacterial agents, Eysenhardtia adenostylis extract or isoflavan compounds will be different according to use, forms, mixing purposes and the like, but generally, 0.00001-20 mass% of the total amount of compositions is preferable. More preferably, 0.0001-10 mass%.

Preparation of antibacterial compositions such as cosmetics including quasi-drugs, pharmaceuticals and foods, with which antibacterial agents, Eysenhardtia adenostylis extract or isoflavan compounds are mixed, is prepared by mixing ingredients generally used in cosmetics, pharmaceuticals, foods and the like within the range where the effects of the present invention are not spoiled.

For example, cosmetics include, though materials contained in ingredients are doubly stated, for example, oil contents, surfactants, moisturizers, polyhydric alcohols, thickeners, water-soluble polymers, film formers, non water-soluble polymers, polymer emulsions, powers, pigments, dyes, lake pigments, lower alcohols, sugars, ultraviolet absorbers, amino acids, vitamins, skin whitening agents, skin activators, blood circulation accelerators, antiseborrheic agents, drugs such as anti-inflammatory (antiphlogistine) and the like, botanical extract, organic acids, organic amines, sequestering agents, pH adjusters, antioxidants, astringent agents, fresheners, fragrances, water, and the like.

Followingly, pharmaceuticals include various supplemental components, though materials contained in ingredients are doubly stated, for example, vehicles, stabilizers, moisturizer, emulsifiers, absorption promoters, pH adjusters, surfactants, diluents, carriers, dissolution aids, flavoring substances, preservatives, fragrances, colorants, coating agents, and the like.

Followingly, foods include various supplemental components, though materials contained in ingredients are doubly stated, for example, sweeteners, acidifiers, preservatives, fragrances, colorants, vehicles, stabilizers, moisturizers, emulsifiers, absorption promoters, pH adjusters, surfactants, diluents, carriers, water, and the like.

Said compositions in the present invention can be prepared in various forms.

For example, cosmetics include, for example, skin care products such as lotions, emulsions, creams, gels, essences, facial masks, and the like; makeups such as face powders, dusting powders, makeup bases, foundations, lipsticks, cheek colors, eyeliners, mascaras, eye shadows, eyebrow pencils, and the like; sun care products such as sunscreen creams, sunscreen lotions, sunscreen oils, carmine lotions, and the like; body care products such as hand care products, antiperspirants, and the like; cleaning agents such as face washes, body shampoos, shampoos, soaps, and the like; hair care products such as rinses, hair treatments, styling gels and waxes, hair creams, hair oils, styling spritz, and the like; and hair growth formula and the like.

Also, formulation of cosmetics can be of a wide range such as solution type, solubilized type, emulsion type, powder type, powder dispersion type, oil solution type, gel type, ointment type, aerosol type, water-oil bilayer type, water-oil-powder three-layer type, and the like.

Also, pharmaceuticals include oral agents, external preparations, injection products, inhalers, nasal and eye drops, and the like. These can be formulated into dosage forms such as tablets, liquids, injection products, ointments, creams, lotions, aerosols, suppositories, and the like, according to use.

Also, food include oral compositions such as chewing gum and candy, fish cake including fish sausages and the like, livestock products including sausages, ham and the like, Japanese and Western confectionaries, noodles, seasonings including sauces and soy sauces, pickles, prepared food, general beverages including soft drinks.

The compositions included in the present invention can be prepared in any form by conventional means.

### Examples

Hereinafter, the present invention is concretely explained by referring to samples tested as examples of specific activity and effects. If not specified, mixing ratios of the compounds are expressed in mass%.

### [Example 1]Antimicrobial activity

Antimicrobial activity was assayed using the method described below. Paraben was used as a positive control.

### [Sample Preparation]

### (Preparation of Eysenhardtia adenostylis extract)

Extraction was performed by adding 300g of chopped dry Eysenhardtia adenostylis xylem to 6L of ethanol, which were left at room temperature over night. Next, extraction was performed by separating the solids from the extract by filtration and newly adding 6L of ethanol to the solids, which were left at room temperature over night. 24g of Eysenhardtia adenostylis extract was obtained by removing the solids from the second extract, which was mixed with the first extract and dried under reduced pressure.

### (Preparation of amorphaquinone, mucronulatol, lonchocarpin and pendulone)

350mg of dry solids in the lower layer was obtained by separation and fractionation adding a proper quantity of chloroform-methanol-water (4:4:3) to 1.56g of dry solids extracted from 20g of dried Eysenhardtia adenostylis wood through the same preparation procedure of said Eysenhardtia adenostylis extract. This dry solids, in order to remove impurities which were absorbed to silica gel, was applied to a silica gel column (2cm x 40cm, solvent; chloroform:methanol=10:1), from which 277mg of dry solids was obtained by collecting 150mL of elute from the beginning of the elution. A single dissolution peak appeared when this was applied to a 2cm × 40cm silica gel column (chloroform solvent), from which 33.2mg of dry solids was obtained (Fraction I). Moreover, the elution was continued changing the solvent composition to chloroform:ethyl acetate=20:1, from which 99.5mg of dry solids was obtained from the first dissolution peak (Fraction II). Three dissolution peaks appeared when Fraction II was applied to a 2cm × 40cm silica gel column (solvent; hexane:ethyl acetate=5:1), from each of which 39.6mg (Fraction II-1), 23.4mg (Fraction II-2), 44.0mg (Fraction II-3) of dry solids were obtained respectively by the order of elution.

It was confirmed that each fraction obtained hitherto, which has been separated using thin layer chromatography with various developing solvents almost aiways showed as a single spot. Also, the ¹H NMR(400MHz), ¹³C NMR(100MHz), EIMS, HREIMS, UV and IR spectra for each fraction were measured, as a result (described below) of which it was identified that the component of Fraction I is amorphaquinone (CAS No. 70283-29-3) (Compound I), Fraction II-1 is mucronulatol (CAS No. 20878-98-2) (Compound II-1), Fraction II-2 is lonchocarpin (CAS No. 23531-99-9) (Compound II-2) and Fraction II-3 is pendulone (CAS No. 69359-09-7) (Compound II-3). Also, these compounds which were obtained were all racemic forms.

### (Fraction I)

HR EI-MS(pos.)m/z:Found 346.1059(Calcd. for C₁₈H₁₈O₇ 346.1053). EI-MS m/z (intensity): 346(31), 256(21), 194(31), 149(81), 137(46), 95(55), 81(100). [α]_{D}(24deg.)in MeOH: 0° (c1.00, racemate). UV(25deg.)λₘₐₓ in MeOH nm(E): 210(16700), 268 (5500), 327(600), 376(400). IRvₘₐₓ in KBR cm⁻¹: 3600-3100, 2938, 1652, 1601, 1527, 1498, 1459, 1349, 1318, 1293, 1200, 1172, 1091, 1045, 987, 895, 850, 802, 669. ¹H NMR(400MHz, in DMSO-d₆)δ: 6.61(1H, d, J= 8.4Hz, 5-H), 6.44(1H, br. s, 6'-H), 6.37(1H, d, J=8.4Hz, 6-H), 4.24(1H, br. d, J=11Hz, 2-Ha), 3.95(1H, dd, J=11, 8.4Hz, 2-Hb), 3.91, 3.89 (each 3H, s. 3'-OCH₃, 4'-OCH₃), 3.65(3H, s, 8-OCH₃), 3.20-3.26(1 H, m, 3-H), 2.82(1H, dd, J=16, 5.2Hz, 4-Ha), 2.73(1H, dd, J=16, 8.8Hz, 4-Hb). ¹³C NMR(100MHz, in DMSO-d₆)δ_{C}: 183.67(s, C-2'), 182.84(s. C-5'), 148.83(s, C-7), 147.33(s, C-8a), 146.16(s, C-1'), 145.24(s, C-4'), 144.38(s, C-3'), 135. 54(s, C-8), 130.29(d, C-6'), 129.54(d, C-5), 112.53(d, C-6), 108.67(s, C-4a), 67.78(t, C-2), 60. 77, 60.68(each q, 3'-OCH₃, 4'-OCH₃), 59.79 (q, 8-OCH₃), 30.26(d, C-3), 28.80(t, C-4)

### (Fraction II-1)

HR EI-MS(pos.)m/z:Found 302.1158(Caicd. for C₁₇H₁₈O₅ 302.1154). EI-MS m/z (intensity) : 302(97). 285(10), 256(19), 180(100), 168(90), 167(92), 149(81), 137(52), 95(43), 81 (88), 69(89), 60(74). [α]_{D}(25deg.) in MeOH: 0°(c1.20, racemate). UV(25deg. )λₘₐₓ in MeOH nm(ε): 206 (18300), 282(1700). IRvₘₐₓ in KBR cm⁻¹: 3600-3100, 2935, 2837, 1618, 1597, 1508, 1463, 1431, 1315, 1294, 1279, 1220, 1157, 1116, 1095, 1025, 967, 941, 844, 792, 735, 701, 627. ¹HNMR (400MHz, in DMSO-d₆)δ: 6.86(1H, d, J=8.4Hz, 5-H), 6.77(1H, d, J=8.8Hz, 5'-H), 6.46(1H, d, J=8.8Hz, 6'-H), 6.28(1H, dd, J=8.4, 2.4Hz, 6-H), 6.18(1 H, d, J=2.4Hz, 8-H).4.15(1H, br.d, J=11Hz, 2-Ha), 3.92(1H, t-like, J=ca. 11 Hz, 2-Hb). 3.74, 3.69(each 3H, s. 2'-OCH₃, 4'-OCH₃), 3.30-3.40 (1 H, m, 3-H), 2.86(1H, dd, J=16,10.8Hz. 4-Ha), 2.71(1H, dd, J=16, 3.6Hz, 4-Hb). ¹³C NMR10OMHz, in DMSO-d₆) δ_{C}: 158.37(s, C-7), 154.50(s, C-8a), 151.51(S, C-2'), 148.02(s, C-4'), 136.12(s, C-1'), 129.94(d, C-5), 121.30(s, C-3'), 121.00(d, C-5'), 112.59(s, C-4a), 107.89(d, C-6'), 103.20(d, C-6), 102.46(d,C-8), 69.05(t, C-2), 60.12 (q, 2'-OCH₃), 54.55 (q, 4' -OCH₃), 31.43(d, C-3), 29.67(t, C-4).

### (Fraction II-2)

HR EI-MS(pos.)m/z:Found 332.1155(Calcd. for C₁₈H₂₀O₆ 332.1260). EI-MS m/z (intensity): 332(97), 210(100), 198(38), 163(22), 149(31), 81(62), 69(84). [α]_{D}(25deg.)in MeOH: 0° (c1.20, racemate). UV(25deg. )λₘₐₓ in MeOH nm(ε): 204 (19200), 288(2600). IRvₘₐₓ in KBR cm⁻¹: 3600-3100, 2938, 2839, 1622, 1596, 1506, 1463, 1435, 1421, 1361, 1277, 1192, 1157, 1115, 1077, 1028, 989, 912, 842, 804, 736. 1H NMR(400MHz, in DMSO-d₆)δ: 6.86 (1 H, d, J=8.0Hz, 5-H), 6.55(1 H, s, 5'-H), 6.28(1 H, dd, J=8.0, 2.4Hz, 6-H), 6.18(1H, d, J=2. 4Hz, 8-H),4.14(1H, br. d, J=11Hz, 2-Ha), 3.97(1H, t-like, J=ca. 11Hz, 2-Hb), 3.75, 3.74(each 3H, s, 2'-OCH₃, 6'-OCH₃), 3.68 (3H, s, 3'-OCH₃), 3.35-3.42(1H, m, 3-H), 2.93(1 H, dd, J=15, 12Hz, 4-Ha), 2.72(1 H, dd, J=15, 4Hz,4-Hb). ¹³C NMR(100MHz, in DMSO-d₆)δ_{C}: 158.38(s, C-7), 154.48(s, C-8a), 151.51 (s, C-6'), 141.90(s, C-2'), 141.25(s, C-4'), 141.15(s, C-3'), 129.92(d, C-5), 122.41(s,C-1'), 112.56(s, C-4a), 107.91 (d, C-6), 106.94(d, C-5'), 102.45(d, C-8), 68.86(t, C-2), 60.69, 60.29 (each q, 2'-OCH₃, 6'-OCH₃), 56.42(q, 3'-OCH₃), 31.73 (d, C-3), 29.64(t, C-4).

### (Fraction II-3)

HR EI-MS(pos.)m/z:Found 316.0946(Calcd. for C₁₇H₁₆O₆ 316.0947). EI-MS m/z (intensity): 316(100), 301(57), 286(22), 283(20), 194(36), 149(35), 69(52). [α]_{D}(24deg.)in MeOH: 0° (c1.50, racemate). UV(25deg.)λₘₐₓ in MeOH nm(ε): 204 (17600), 268(5900), 332(500), 386(350). IRvₘₐₓ in KBR cm⁻¹: 3700-3100, 2960, 1652, 1601, 1509, 1456, 1317, 1296, 1277, 1219, 1155, 1118, 1066, 1036, 1007, 984, 943, 893, 846, 801, 736. ¹H NMR (400MHz, in DMSO-d₆)δ: 6.86(1 H, d, J=8.4Hz, 5-H), 6.43(1 H, s, 6'-H), 6.31 (1 H, dd, J=8. 4. 2.4Hz, 6-H),6.18(1H, d, J=2. 4Hz, 8-H),4.16(1H, br. d, J=11Hz, 2-Ha), 3.91, 3.89(each 3H, s, 3'-OCH₃, 4'-OCH₃), 3.70-3.85(1H, m, 2-Hb), 3.18-3.24(1H, m, 3-H), 2.81 (1 H, dd, J=16, 5.2Hz, 4-Ha), 2.71(1H, dd, J=16, 8. 8Hz, 4-Hb). ¹³C NMR(100MHz in DMSO-d₆)δ_{C}: 183.68 (s, C-2'), 182.86(s, C-5'), 158.60(s,C-7), 154.14(s, C-8a), 146.20(s, C-1'), 145.23(s, C-4'), 144.38(s, C-3'), 130.27(d, C-6'), 129.97(d, C-5), 111.19(s, C-4a), 108.38(d, C-6), 102.52(d, C-8), 67.73(t, C-2), 60.67, 60.78(each q, 3'-OCH₃, 4'-OCH₃), 30.47(d, C-3), 28.60(t, C-4).

### [Tested Strains]

Staphylococcus aureus (IFO13276 ATCC6538), Candida albicans (IFO1594 ATCC10231), Aspergillus niger (IFO9455 ATCC16401), Pseudomonas aeruginosa (IFO13275 ATCC9027), Escherichia coli (IFO3972 ATCC8739)

### [Evaluation Method]

Antibacterial effect was evaluated by using either qualitative observation of the growing strains in the presence of each specimen or measuring the minimum inhibitory concentration (MIC) of the strains. An agar medium dilution method was adopted for the evaluation using Mueller-Hinton agar media (Eiken Chemical Co., Ltd.). The experiment was performed using the MIC test method following the standards of the Japanese Society of Chemotherapy. More specifically, each specimen was measured, when dissolved in 50/50 ethanol/water mixture and used to prepare 1 mL of each test specimen. 9mL of measuring medium was added to the test specimens to create an agar plate. 50µg of the experimental bacterial liquid (live bacteria: 10⁶-10⁸/mL) was added to the agar plate. The growth and development of the bacteria was judged and recorded after it was cultured for 24 hours at 37°C. The minimum concentration that inhibited bacterial growth was regarded as MIC.

The evaluation results of Example 1 are shown in Table 1 (MIC Rate) and Table 2 (Antibacterial Effect with Specimen Concentration). The MIC rates in Table 1 are given in µg/ml. The more active the antibacterial effect, the lower the rate becomes. The meaning of the symbols used in Table 2 is as follows: A: antibacterially effective, B: bacteriostatically active, C: some growth inhibiting effective, D: non effective.

**[Table 1] MIC Rate**

| Strain | Eysenhardtia adenostylis extract | Paraben |
|---|---|---|
| Staphylococcus aureus | 100 | 800 |
| Pseudomonas aeruginosa | 500 | 1000 |
| Escherichia coli | 700 | 800 |
| Candida albicans | 300 | 500 |
| Aspergillus niger | 1000 | 300 |

**[Table 2] Antibacterial Effect with Specimen Concentration**

| Specimen: Amorphaquinone | | |
|---|---|---|
| Strain | Specimen concentration | |
| | 500µg/ml | 50µg/ml |
| Staphylococcus aureus | A | A |
| Candida albicans | A | B |
| Aspergillus niger | A | C |
| | | |

| Specimen: Mucronulatol | | |
|---|---|---|
| Strain | Specimen concentration | |
| | 500µg/ml | 50µg/ml |
| Staphylococcus aureus | A | A |
| Candida albicans | A | C |
| Aspergillus niger | A | C |
| | | |

| Specimen: Lonchocarpin | | |
|---|---|---|
| Strain | Specimen | concentration |
| | 500µg/ml | 50µg/ml |
| Staphylococcus aureus | C | D |
| Candida albicans | C | C |
| Aspergillus niger | A | C |
| | | |

| Specimen: Pendulone | | |
|---|---|---|
| Strain | Specimen | concentration, |
| | 500µg/ml | 50µg/ml |
| Staphylococcus aureus | A | A |
| Candida albicans | A | C |
| Aspergillus niger | A | C |
| | | |

| Specimen: Liquid solution only (control) | | |
|---|---|---|
| Strain | Specimen concentration | |
| | The same amount as the above tests | |
| Staphylococcus aureus | D | |
| Candida albicans | D | |
| Aspergillus niger | D | |

As seen in tables 1 and 2, Eysenhardtia adenostylis extract, amorphaquinone, mucronulatol, lonchocarpin and pendulone all expressed significant antibacterial activity in relation to microbes and fungi. The results show Eysenhardtia adenostylis extract, amorphaquinone, mucronulatol, lonchocarpin and pendulone will serve as potent antibacterial agents.

The examples of the antibacterial composition of this invention are as follows:

### [Example 2] Lotion

| Ingredient | Blending quantity (mass%) |
|---|---|
| Polyoxyethylene(12) polyoxypropylene(6) tetradecylethyl | 1.0 |
| Dipropylene glycol | 4.0 |
| Glycerin | 4.0 |
| 1,3-butylene glycol | 2.0 |
| Oleyl alcohol | 0.1 |
| Ethanol | 13.0 |
| Citric acid | 0.1 |
| Sodium citrate | 0.3 |
| Pendulone | 0.5 |
| Fragrance | proper quantity |
| Purified water | remaining quantity |

### [Example 3] Emulsion

| Ingredient | Blending quantity (mass%) |
|---|---|
| Stearic acid | 2.0 |
| Catanol | 0.5 |
| Liquid paraffin | 10.0 |
| Liquid lanolin | 6.0 |
| Squalan | 5.0 |
| Glycerol tris | 1.0 |
| Sorbitan mono-oleate ester | 2.0 |
| Glycerin | 6.0 |
| Triethanolamine | 0.6 |
| Purified water | remaining quantity |
| Eysenhardtia adenostyiis extract | 0.1 |
| Fragrance | proper quantity |

### [Example 4] Hair treatment lotion

| Ingredient | Blending quantity (mass%) |
|---|---|
| Dipropylene glycol | 2.0 |
| 1,3-butylene glycol | 1.0 |
| Glycerin | 1.0 |
| Stearyl trimethyl ammoium chloride | 0.5 |
| Methylphenylpolysiloxane | 1.0 |
| Collagen hydrolysate | 1.0 |
| Ethanol | 50.0 |
| Purified water | remaining quantity |
| Fragrance | proper quantity |

### [Example 5] Shampoo

| Ingredient | Blending quantity (mass%) |
|---|---|
| Polyoxyethylene(3) triethanolamine laureth sulfate | 12.0 |
| Sodium laureth sulfate | 4.5 |
| Lauryldiethanolamid | 3.0 |
| Ethylene glycol distearate | 2.0 |
| Lanoline derivative | 1.0 |
| Citric acid | 0.1 |
| Mucronulatol | 0.1 |
| Fragrance | proper quantity |
| Purified water | remaining quantity |

### [Example 6] Cream

| Ingredient | Blending quantity (mass%) |
|---|---|
| Cetyl alcohol | 5.0 |
| Stearic acid | 2.0 |
| Hydrogenated lanoline | 4.0 |
| Squalan | 9.0 |
| Octyldodecanol | 10.0 |
| Glycerin | 4.0 |
| Dipropylene glycol | 3.0 |
| Polyoxyethylene(25) cetyl ether | 3.0 |
| Glyceral monostearate | 2.0 |
| Purified water | remaining quantity |
| Mucronulatol | 0.005 |
| Antioxidant | proper quantity |
| Fragrance | proper quantity |

### [Example 7] Mascara

| Ingredient | Blending quantity (mass%) |
|---|---|
| Polyacrylic ester emulsion (Polymer concentration 50 mass%) | 38.0 |
| 1,3-butylene glycol | 3.0 |
| Black iron oxide | 8.0 |
| Triethanolamine | 1.0 |
| Purified water | remaining quantity |
| Eysenhardtia adenostylis extract | 0.5 |
| Fragrance | proper quantity |

### [Example 8] Mascara

| Ingredient | Blending quantity (mass%) |
|---|---|
| Polyacrylic ester emulsion (Polymer concentration 50 mass%) | 38.0 |
| 1,3-butylene glycol | 3.0 |
| Black iron oxide | 8.0 |
| Triethanolamine | 1.0 |
| Purified water | remaining quantity |
| Pendulone | 0.5 |
| Fragrance | proper quantity |

### [Example 9] Face wash

| Ingredient | Blending quantity (mass%) |
|---|---|
| Stearic acid | 10.0 |
| Palmitic acid | 10.0 |
| Myristic acid | 12.0 |
| Lauric acid | 4.0 |
| Palm oil | 2.0 |
| Potassium hydroxide | 6.0 |
| PEG1500 | 10.0 |
| Glycerin | 15.0 |
| Glyceral monostearate | 2.0 |
| Polyoxyethylene(20) sorbitan mono-stearate | 2.0 |
| Purified water | remaining quantity |
| Fragrance | proper quantity |
| amorphaquinone | 0.5 |

### [Example 10] Hair cream

| Ingredient | Blending quantity (mass%) |
|---|---|
| Bee wax | 3.0 |
| Liquid paraffin | 15.0 |
| Squalan | 5.0 |
| Microcrystallen wax | 5.0 |
| Behenyl alcohol | 1.0 |
| Glycerol tris | 8.0 |
| Polyoxyethylene(20) behenyl ether | 2.0 |
| Polyoxyethylene(40) sorbit tetraoleate | 1.0 |
| Glyceral monostearate | 2.0 |
| 1,3-butylene glycol | 5.0 |
| Glycerin | 2.0 |
| Pendulone | 0.1 |
| Purified water | remaining quantity |
| Fragrance | proper quantity |

### [Example 11] Face cream

| Ingredient | Blending quantity (mass%) |
|---|---|
| Myristic acid | 10.0 |
| Stearic acid | 8.0 |
| Palmitic acid | 5.0 |
| Lauric acid | 2.0 |
| Sodium laureth sulfate | 8.0 |
| Lauric acid diethanolamine | 3.0 |
| Lanolin | 1.5 |
| Catanol | 3.0 |
| Potassium hydroxide | 6.0 |
| Glycerin | 15.0 |
| Mucronulatol | 0.5 |
| Purified water | remaining quantity |
| Fragrance | proper quantity |

### [Example 12] Acne lotion

| Ingredient | Blending quantity (mass%) |
|---|---|
| Sorbit | 2.0 |
| 1,3-butylene glycol | 2.0 |
| PEG1000 | 1.0 |
| Polyoxyethylene(25) oleylether | 1.0 |
| Ethanol | 10.0 |
| Lonchocarpin | 0.5 |
| Purified water | remaining quantity |

### [Example 13] Antiperspirant (roll-on formulation)

| Ingredient | Blending quantity (mass%) |
|---|---|
| Light liquid Isoparaffin | 5.0 |
| Cetostearyl alcohol | 0.8 |
| Polyethyleneglycol monostearate(40) | 5.0 |
| 1,3-butylene glycol | 2.0 |
| Magnesium aluminum silicate | 0.8 |
| Aluminum chlorohydrate (50% ethanol solution) | 40.0 |
| Eysenhardtia adenostylis extract | 0.8 |
| Fragrance | proper quantity |
| Purified water | remaining quantity |

### [Example 14] Hair treatment lotion

| Ingredient | Blending quantity (mass%) |
|---|---|
| 1,3-butylene glycol | 2.0 |
| Glycerin | 1.0 |
| Stearyl trimethyl ammoium chloride | 0.5 |
| Methylphenylpolysiloxane | 1.0 |
| Collagen hydrolysate | 1.0 |
| Ethanol | 50.0 |
| Purified water | remaining quantity |
| Fragrance | proper quantity |
| Amorphaquinone | 0.6 |

### [Example 15] Sausage

| Ingredient | Blending quantity (mass%) |
|---|---|
| Beef | 88.7 |
| Chicken egg | 5.0 |
| Condiment | 0.3 |
| Glutamic sodium | 0.5 |
| Salt | remaining quantity |
| Lonchocarpin | 0.5 |

### [Example 16] Sausage

| Ingredient | Blending quantity (mass%) |
|---|---|
| Beef | 88.7 |
| Chicken egg | 5.0 |
| Condiment | 0.3 |
| Glutamic sodium | 0.5 |
| Salt | remaining quantity |
| Eysenhardtia adenostylis extract | 0.5 |

### [Example 17] Lotion

| Ingredient | Blending quantity (mass%) |
|---|---|
| 1,3-butylene glycol | 6.0 |
| Glycerin | 4.0 |
| Oleyl alcohol | 0.1 |
| Polyoxyethylene(20) Sorbitan mono-oleate ester | 0.5 |
| Polyoxyethylene(15) Lauryl alcohol ether | 0.5 |
| Ethanol | 10.0 |
| Mucronulatol | 0.2 |
| Fragrance | proper quantity |
| Purified water | remaining quantity |

### [Example 18] Emulsion

| Ingredient | Blending quantity (mass%) |
|---|---|
| Stearic acid | 2.0 |
| Catanol | 1.5 |
| Petrolatum | 4.0 |
| Squalan | 5.0 |
| Glycerol tris | 2.0 |
| Sorbitan mono-oleate ester | 2.0 |
| 1,3-butylene glycol | 3.0 |
| Glycerin | 2.0 |
| PEG 1500 | 3.0 |
| Triethanolamine | 1.0 |
| Purified water | remaining quantity |
| Lonchocarpin | 1.0 |
| Fragrance | proper quantity |

### [Example 19] Hair treatment lotion

| Ingredient | Blending quantity (mass%) |
|---|---|
| 1,3-butylene glycol | 2.0 |
| Glycerin | 1.0 |
| Stearyl trimethyl ammoium chloride | 0.5 |
| Methylphenylpolysiloxane | 1.0 |
| Collagen hydrolysate | 1.0 |
| Ethanol | 50.0 |
| Purified water | remaining quantity |
| Fragrance | proper quantity |
| Eysenhardtia adenostylis extract | 0.6 |

### [Example 20] Cream

| Ingredient | Blending quantity (mass%) |
|---|---|
| Stearyl alcohol | 6.0 |
| Stearic acid | 2.0 |
| Hydrogenated lanoline | 4.0 |
| Squalan | 9.0 |
| Octyldodecanol | 10.0 |
| 1,3-butylene glycol | 6.0 |
| PEG1500 | 4.0 |
| Polyoxyethylene(25) cetyl alcohol ether | 3.0 |
| Glyceral monostearate | 2.0 |
| Purified water | remaining quantity |
| Amorphaquinone | 0.005 |
| Antioxidant | proper quantity |
| Fragrance | proper quantity |

### [Example 21] Sausage

| Ingredient | Blending quantity (mass%) |
|---|---|
| Beef | 88.7 |
| Chicken egg | 5.0 |
| Condiment | 0.3 |
| Glutamic sodium | 0.5 |
| Salt | remaining quantity |
| Mucronulatol | 0.5 |

### [Example 22] Shampoo

| Ingredient | Blending quantity (mass%) |
|---|---|
| Polyoxyethylene(3) triethanolamine laureth sulfate | 12.0 |
| Triethanolamine laureth sulfate | 6.0 |
| Lauryldiethanolamid | 6.0 |
| Sodium chloride | 0.1 |
| Citric acid | 0.1 |
| Pendulone | 0.1 |
| Fragrance | proper quantity |
| Purified water | remaining quantity |

### [Example 23] Acne ointment

| Ingredient | Blending quantity (mass%) |
|---|---|
| Stearic acid | 15.0 |
| Catanol | 1.0 |
| Potassium hydroxide (10 mass% solution) | 7.0 |
| Glycerin | 3.0 |
| Dipropylene glycol | 5.0 |
| Purified water | remaining quantity |
| Amorphaquinone | 1.0 |

### [Example 24] Cream

| Ingredient | Blending quantity (mass%) |
|---|---|
| Stearyl alcohol | 6.0 |
| Stearic acid | 2.0 |
| Hydrogenated lanoline | 4.0 |
| Squalan | 9.0 |
| Octyldodecanol | 10.0 |
| 1,3-butylene glycol | 6.0 |
| PEG1500 | 4.0 |
| Polyoxyethylene(25)cetyl alcohol ether | 3.0 |
| Glyceral monostearate | 2.0 |
| Purified water | remaining quantity |
| Pendulone | 0.005 |
| Antioxidant | proper quantity |
| Fragrance | proper quantity |

### [Example 25] Mascara

| Ingredient | Blending quantity (mass%) |
|---|---|
| Iron oxide (black) | 10.0 |
| Poiyacryiic ester emulsion (Polymer concentration 50 mass%) | 30.0 |
| Solid paraffin | 8.0 |
| Lanolin wax | 8.0 |
| Light isoparaffin | 30.0 |
| Sorbitan sesquiolete | 4.0 |
| Purified water | remaining quantity |
| Amorphaquinone | 0.1 |
| Fragrance | proper quantity |

### [Example 26] Mascara

| Ingredient | Blending quantity (mass%) |
|---|---|
| Iron oxide (black) | 10.0 |
| Polyacrylic ester emulsion (Polymer concentration 50 mass%) | 30.0 |
| Solid paraffin | 8.0 |
| Lanolin wax | 8.0 |
| Light isoparaffin | 30.3 |
| Sorbitan sesquiolete | 4.0 |
| Purified water | remaining quantity |
| Mucronulatol | 0.1 |
| Fragrance | proper quantity |

### [Example 27] Mascara

| Ingredient | Blending quantity (mass%) |
|---|---|
| Iron oxide (black) | 10.0 |
| Polyacrylic ester emulsion (Polymer concentration 50 mass%) | 30.0 |
| Solid paraffin | 8.0 |
| Lanolin wax | 8.0 |
| Light isoparaffin | 30.0 |
| Sorbitan sesquiolete | 4.0 |
| Purified water | remaining quantity |
| Lonchocarpin | 0.1 |
| Fragrance | proper quantity |

### [Example 28] Hair Rinse

| Ingredient | Blending quantity (mass%) |
|---|---|
| Silicone oil | 3.0 |
| Liquid paraffin | 1.0 |
| Catanol | 1.5 |
| Stearyl alcohol | 1.0 |
| Stearyl trimethyl ammoium chloride | 0.7 |
| Glycerin | 3.0 |
| Pigment | proper quantity |
| Eysenhardtia adenostylis extract | 0.02 |
| Fragrance | proper quantity |
| Purified water | remaining quantity |

### [Example 29] Hair cream

| Ingredient | Blending quantity (mass%) |
|---|---|
| Bee wax | 3.0 |
| Liquid paraffin | 20.0 |
| Microcrystallen wax | 5.0 |
| Behenyl alcohol | 1.3 |
| Cetyl 2-ethylhexanoate | 10.0 |
| Polyoxyethylene(20) behenyl ether | 2.0 |
| Polyoxyethylene(40) sorbit tetraoleate | 1.0 |
| Glyceral monostearate | 2.0 |
| 1,3-butylene glycol | 5.0 |
| Mucronulatol | 0.1 |
| Purified water | remaining quantity |
| Fragrance | proper quantity |

### [Example 30] Hair rinse

| Ingredient | Blending quantity (mass%) |
|---|---|
| Silicone oil | 0.5 |
| Catanol | 2.0 |
| Stearyl alcohol | 1.0 |
| Octyldodecanol | 1.0 |
| Stearyl trimethyl ammoium chloride | 3.0 |
| Dimethyldistearylammonium Chloride | 3.0 |
| Polyoxyethylene(2) oleylether | 2.0 |
| Cationized cellulose (10 mass% solution) | 2.0 |
| Dipropylene glycol | 3.0 |
| Lonchocarpin | 0.02 |
| Pigment | proper quantity |
| Fragrance | proper quantity |
| Purified water | remaining quantity |

### [Example 31] Body shampoo

| Ingredient | Blending quantity (mass%) |
|---|---|
| Lauric acid | 2.5 |
| Myristic acid | 7.5 |
| Palmitic acid | 2.5 |
| Oleic acid | 2.5 |
| Lauryldiethanolamid | 5.0 |
| Glycerin | 20.0 |
| Potassium hydroxide | 3.6 |
| Purified water | remaining quantity |
| Amorphaquinone | 0.2 |
| Fragrance | proper quantity |

### [Example 32] Acne lotion

| Ingredient | Blending quantity (mass%) |
|---|---|
| Sorbit | 2.0 |
| 1,3-butylene glycol | 2.0 |
| PEG1000 | 1.0 |
| Polyoxyethylene(25) oleylether | 1.0 |
| Ethanol | 10.0 |
| Eysenhardtia adenostylis extract | 5.0 |
| Purified water | remaining quantity |

### [Example 33] Antiperspirant

| Ingredient | Blending quantity (mass%) |
|---|---|
| Stearic acid | 8.0 |
| Ethanol | 22.0 |
| Sorbitol | 4.2 |
| Silicic anhydride | 0.8 |
| Sodium hydroxide | 1.2 |
| Purified water | remaining quantity |
| Aluminum chlorohydrate (50% ethanol solution) | 40.0 |
| Mucronulatol | 0.8 |

### [Example 34] Antiperspirant

| Ingredient | Blending quantity (mass%) |
|---|---|
| Stearic acid | 8.0 |
| Ethanol | 22.0 |
| Sorbitol | 4.2 |
| Silicic anhydride | 0.8 |
| Sodium hydroxide | 1.2 |
| Purified water | remaining quantity |
| Aluminum chlorohydrate (50% ethanol solution) | 40.0 |
| Lonchocarpin | 0.8 |

### [Example 35] Antiperspirant

| Ingredient | Blending quantity (mass%) |
|---|---|
| Stearic acid | 8.0 |
| Ethanol | 22.0 |
| Sorbitol | 4.2 |
| Silicic anhydride | 0.8 |
| Sodium hydroxide | 1.2 |
| Purified water | remaining quantity |
| Aluminum chlorohydrate (50% ethanol solution) | 40.0 |
| Pendulone | 0.8 |

### [Example 36] Sausage

| Ingredient | Blending quantity (mass%) |
|---|---|
| Beef | 88.7 |
| Chicken egg | 5.0 |
| Condiment | 0.3 |
| Glutamic sodium | 0.5 |
| Salt | remaining quantity |
| Pendulone | 0.4 |

### [Example 37] Sausage

| Ingredient | Blending quantity (mass%) |
|---|---|
| Beef | 88.7 |
| Chicken egg | 5.0 |
| Condiment | 0.3 |
| Glutamic sodium | 0.5 |
| Salt | remaining quantity |
| Amorphaquinone | 0.4 |

### Industrial Applicability

Since Eysenhardtia adenostylis extract and the isoflavan compounds of a specific structure that exist in Eysenhardtia adenostylis have antibacterial activity, these can be used as antibacterial agents which are safe for regular use for products that require antibacterial and antiseptic properties such as cosmetics, pharmaceuticals, foods and the like.

## Claims

1. Antibacterial agents comprising Eysenhardtia adenostylis extract.

2. Antibacterial agents containing Eysenhardtia adenostylis extract as an effective ingredient.

3. Antibacterial agents 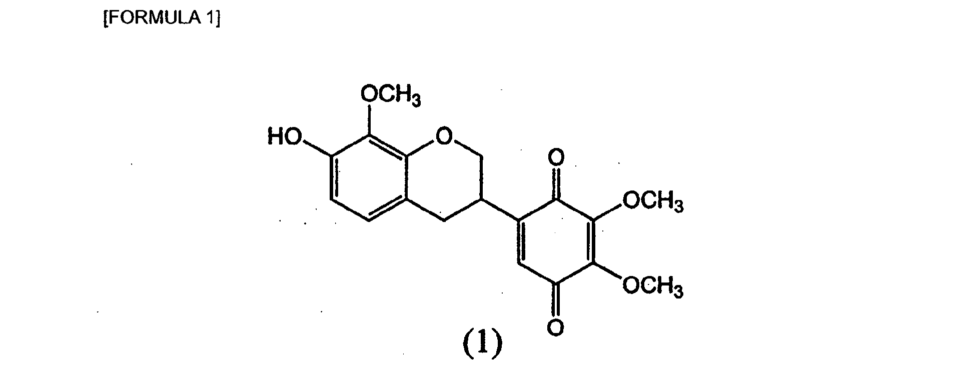 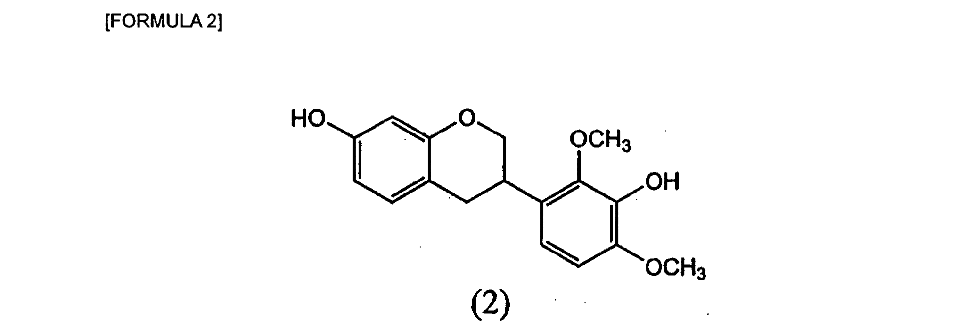 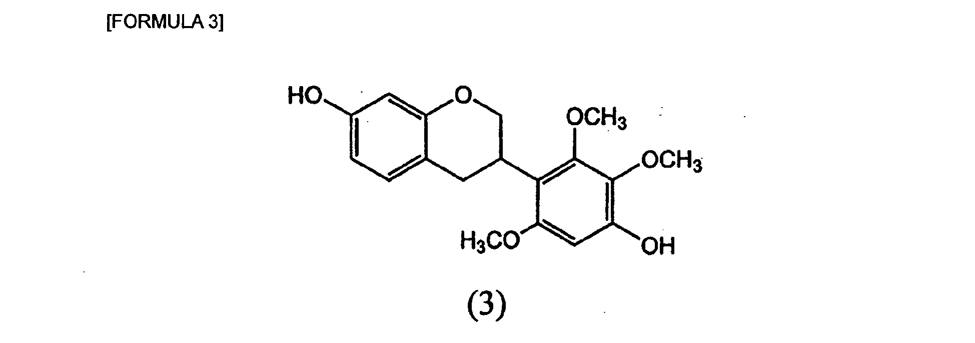 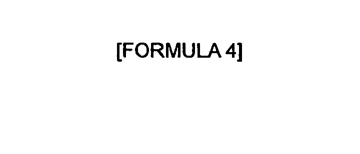 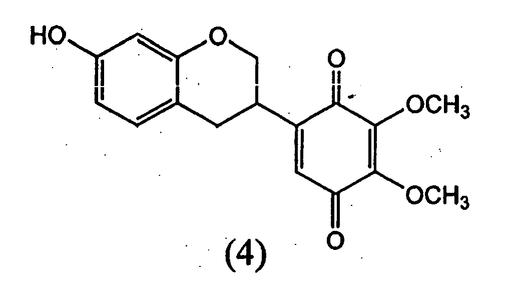 comprising one or more compounds selected from the isoflavan compounds shown in the formulae (1), (2), (3) and (4).

4. Antibacterial agents containing as active components one or more compounds selected from the isoflavan compounds shown in the formulae (1), (2), (3) and (4) in Claim 3.

5. Antibacterial compositions comprising the antibacterial agents claimed in any of claims 1 to 4.

6. Antibacterial compositions comprising Eysenhardtia adenostylis extract.

7. Antibacterial compositions comprising one or more compounds selected from the isoflavan compounds shown in the formulae (1), (2), (3) and (4) in Claim 3.

8. Antibacterial compositions claimed in any of claims 5 to 7 whose compositions are cosmetics, pharmaceuticals or foods.

9. Compositions containing the antibacterial agents claimed in any of claims 1 to 4.

10. Compositions containing Eysenhardtia adenostylis extract.

11. Compositions containing one or more compounds selected from the isoflavan compounds shown in the formulae (1), (2), (3) and (4) in Claim 3.

12. Compositions claimed in any of claims 9 to 11 whose compositions are cosmetics, pharmaceuticals or foods.
